# FASCICULE DE BREVET EUROPEEN

(11) **EP 0 742 234 B1**
(45) Date de publication et mention de la délivrance du brevet: **11.04.2001**
(21) Numéro de dépôt: 96400920.3
(22) Date de dépôt: 29.04.1996
(51) Int. Cl.: C08F 110/10, C08F 2/00, C07C 6/04, C08F 4/12, C08F 4/14

(54) **Procédé et installation pour la conversion de coupes C4 oléfiniques en polyisobutène et en propylène**
Verfahren und Einrichtung zur Umsetzung des olefinischen C4-Schnitts in Polyisobuten und Propylen
Process and installation for the conversion of the olefinic C4-cut into polyisobutene and propylene

(30) Priorité: 11.05.1995 FR 9505560
(43) Date de publication de la demande: 13.11.1996
(73) Titulaire: INSTITUT FRANCAIS DU PETROLE, 92502 Rueil-Malmaison (FR)
(72) Inventeur: Chodorge, Jean-Alain, 92160 Antony (FR); Commereuc, Dominique, 92190 Meudon (FR); Cosyns, Jean,, 78580 Maule (FR); Duee, Didier, 95610 Eragny Sur Oise (FR); Torck, Bernard, 92100 Boulogne Sur Seine (FR)

(56) Documents cités:
- EP-A- 0 213 401
- FR-A- 2 442 246
- FR-A- 2 709 125
- US-A- 3 501 551
- US-A- 5 414 179
- US-A- 5 416 176
- DATABASE WPI Section Ch, Week 7217 Derwent Publications Ltd., London, GB; Class A41, AN 72-27312t XP002011058 & JP-B-47 013 251 (MARUZEN PETRO-CHEMICAL CO)

## Description

L'objet de la présente invention est un procédé de conversion d'une coupe C₄ oléfinique d'une part en polyisobutène et d'autre part en propylène. Une partie, ou la totalité, de la coupe C₄ peut provenir de la conversion d'une coupe C₅ oléfinique. Lorsque ces coupes sont issues d'une opération de vapocraquage, un autre objet de la présente invention est l'optimisation de la sélectivité relative éthylène-propylène du vapocraquage grâce à la mise en oeuvre de ce procédé.

Le vapocraquage de charges constituées par des coupes paraffiniques légères fournit l'éthylène et le propylène nécessaires à la pétrochimie. Il fournit également un certain nombre d'autres produits plus lourds, et en particulier une coupe d'hydrocarbures en C₄ qui contient principalement du butadiène-1,3, de l'isobutène, des n-butènes et des butanes, accompagnés de traces d'hydrocarbures acétyléniques, et une coupe d'hydrocarbures en C₅ qui contient principalement des dioléfines en C₅, des méthylbutènes, des n-pentènes et des pentanes, accompagnés de traces d'hydrocarbures acétyléniques.

Le craquage catalytique, par exemple en lit fluide (FCC), de charges d'hydrocarbures lourds, fournit, à côté des fractions essence et gazole qui sont les produits principaux, des produits plus légers, parmi lesquels une coupe d'hydrocarbures en C₄ qui contient principalement de l'isobutane, de l'isobutène, des n-butènes et des butanes, accompagnés de faibles quantités de butadiène-1,3 et d'hydrocarbures acétyléniques, et une coupe d'hydrocarbures en C₅ qui contient principalement des pentanes, des méthylbutènes et des n-pentènes, accompagnés de faibles quantités de dioléfines en C₅ et d'hydrocarbures acétyléniques.

Jusqu'à récemment, seuls le butadiène-1,3 et l'isobutène trouvaient un usage dans l'industrie des polymères, en particulier dans l'industrie des pneumatiques pour le premier. L'augmentation de la longévité des pneumatiques et une relative stagnation de la demande font qu'il existe maintenant des surplus de butadiène qui ne sont pas, ou mal, utilisés. Au contraire, il y a un regain d'intérêt pour l'isobutène qui peut être utilisé pour la synthèse de polymères ayant des usages multiples.

La présente invention propose un procédé de traitement d'une coupe d'hydrocarbures en C₄ contenant principalement de l'isobutène, des n-butènes, des butanes, et du butadiène-1,3 en quantité variable, qui inclut la transformation de l'isobutène en polyisobutène, et qui permet de transformer le butadiène-1,3 et les n-butènes en propylène utilisable par exemple pour la polymérisation. Une partie, ou la totalité, des hydrocarbures en C₄ peut aussi provenir d'une coupe C₅ oléfinique qui est transformée, après hydroisomérisation, essentiellement en propylène et en un mélange principalement de butène-1 et d'isobutène par métathèse avec l'éthylène.

Les proportions relatives d'éthylène et de propylène produits dans une opération de vapocraquage peuvent être modulées dans une certaine mesure en changeant la nature de la charge et en modifiant les conditions opératoires (la sévérité) du craquage. Cependant, dans un mode de fonctionnement orienté vers une proportion plus grande de propylène, il se fait inévitablement des quantités plus importantes de coupe C₄, de coupe C₅ et de fractions plus lourdes d'essences de mauvaise qualité.

Un autre but de la présente invention est d'optimiser la sélectivité relative éthylène-propylène du vapocraquage grâce au traitement des coupes d'hydrocarbures de vapocraquage en C₄ ou en C₅ qui produit entre autres du propylène, ce qui permet d'ajuster les proportions relatives d'éthylène et de propylène sans être obligé de changer la sévérité du craquage.

Le procédé objet de l'invention est plus précisément un procédé de conversion d'une coupe C₄ oléfinique en polyisobutène et en propylène, ladite coupe pouvant contenir notamment des dioléfines, du butène-1, de l'isobutène et des impuretés acétylèniques, ledit procédé comportant les étapes suivantes se déroulant successivement :
1) hydrogénation sélective des dioléfines et des impuretés acétyléniques avec simultanément isomérisation du butène-1 en butènes-2, par passage de ladite coupe en phase liquide sur un catalyseur comprenant au moins un métal choisi dans le groupe formé par le nickel, le palladium et le platine, déposé sur un support, à une température de 20-200 °C, une pression de 1-5 MPa, une vitesse spatiale de 0,5-10 h⁻¹, avec un rapport H₂ / dioléfines (molaire) de 0,5 à 5 et de préférence de 1 à 3, de façon à obtenir un effluent contenant majoritairement du butène-2 et de l'isobutène, et ne contenant pratiquement pas de dioléfines ni de composés acétyléniques,
2) polymérisation de l'isobutène contenu dans l'effluent de l'étape 1 en polyisobutène, en présence d'un catalyseur acide, à une température de -100 à +100 °C, à une pression telle que la réaction se déroule en phase liquide, une séparation de l'isobutène des autres constituants de la coupe issue de l'étape 1 pouvant avoir lieu avant la polymérisation, pour obtenir d'une part du polyisobutène et d'autre part une coupe C₄ résiduelle sans polymère,
3) métathèse de la coupe C₄ résiduelle avec de l'éthylène, en présence d'un catalyseur comportant au moins un oxyde de rhénium déposé sur un support, à une température comprise entre 0 et 100 °C, et une pression au moins égale à la tension de vapeur du mélange réactionnel à la température de réaction, de façon à obtenir un effluent contenant du propylène, la métathèse étant suivie d'une séparation du propylène.

Dans la coupe C₄ soumise aux traitements selon le procédé ci-dessus, l'isobutène et le butène-1 peuvent être issus en partie, ou en totalité, d'une coupe C₅ oléfinique pouvant contenir notamment des dioléfines, des n-pentènes, des méthylbutènes et des impuretés acétylèniques, qui est soumise à un traitement comportant au moins les deux étapes suivantes se déroulant successivement :
1) hydrogénation sélective des dioléfines et des impuretés acétyléniques avec simultanément isomérisation des oléfines alpha à 5 atomes de carbone en pentènes-2 et en méthyl-2 butène-2, par passage de ladite coupe en phase liquide sur un catalyseur comprenant au moins un métal choisi dans le groupe formé par le nickel, le palladium et le platine, déposé sur un support, à une température de 20-200 °C, une pression de 1-5 MPa, une vitesse spatiale de 0,5-10 h⁻¹, avec un rapport H₂ / dioléfines (molaire) de 0,5 à 5 et de préférence de 1 à 3, de façon à obtenir un effluent contenant majoritairement du pentène-2 et du méthyl-2 butène-2, et ne contenant pratiquement pas de dioléfines ni de composés acétyléniques,
2) métathèse d'au moins une partie de l'effluent issu de l'étape précédente avec de l'éthylène, en présence d'un catalyseur comportant au moins un oxyde de rhénium déposé sur un support, à une température comprise entre 0 et 100 °C, et une pression au moins égale à la tension de vapeur du mélange réactionnel à la température de réaction, de façon à obtenir un effluent contenant principalement du propylène, du butène-1 et de l'isobutène, la métathèse étant suivie d'une séparation du propylène,
3) introduction du mélange butène-1 et isobutène dans l'étape d'hydrogénation sélective de la coupe C₄.

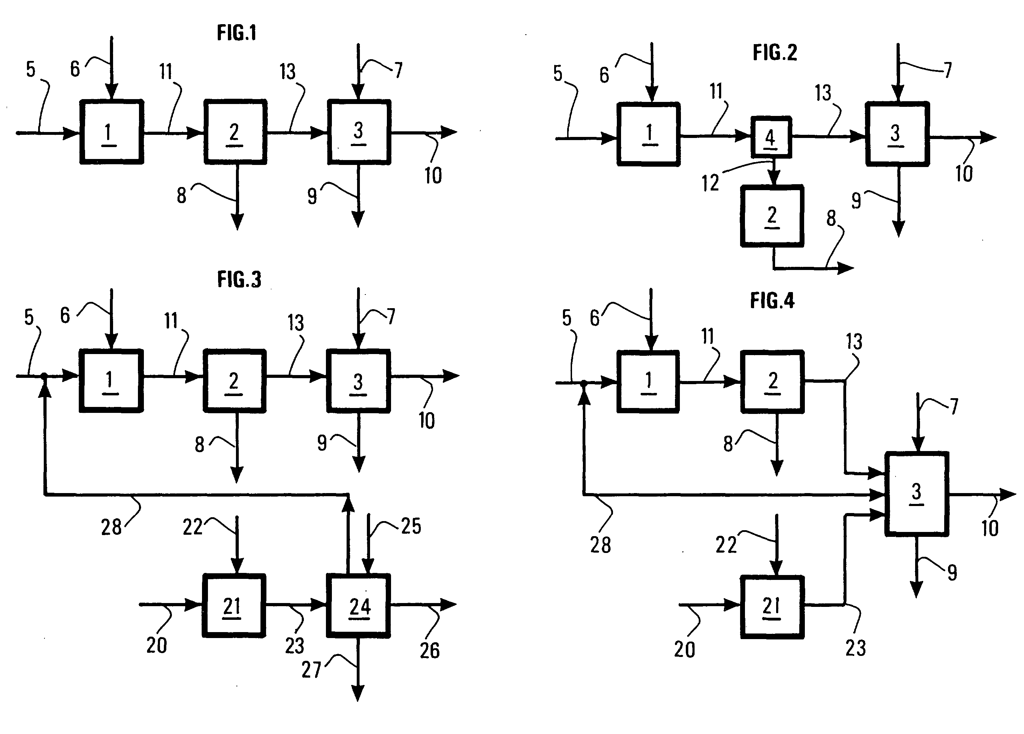

On décrira plus en détail le procédé selon l'invention (illustré figure 1), à partir d'une coupe d'hydrocarbures en C₄ amenée par un conduit 5, qui contient principalement de l'isobutène, des n-butènes, des butanes, ainsi que du butadiène-1,3 en quantité variable. La coupe C₄ est soumise à une succession de traitements regroupés dans les étapes suivantes, pour produire du polyisobutène et du propylène :
- hydroisomérisation sélective du butadiène-1,3 avec isomérisation du butène-1 en butène-2 et hydrogénation des hydrocarbures acétyléniques,
- polymérisation de l'isobutène, éventuellement après une séparation de l'isobutène des autres constituants de la coupe issue de la première étape,
- métathèse de l'effluent riche en butène-2 en présence d'éthylène (éthènolyse) produisant du propylène.

La succession des traitements retenue dans le procédé selon l'invention présente de nombreux avantages. Les composés les plus réactifs de la coupe, à savoir les dioléfines (le butadiène-1,3 par exemple) en quantité variable, ainsi que les traces d'hydrocarbures acétyléniques sont transformés dès la première étape, et ne seront donc pas la cause de réactions parasites dans les étapes suivantes. Par ailleurs l'hydroisomérisation sélective des dioléfines (butadiène-1,3 par exemple) dans les coupes de vapocraquage permet d'augmenter considérablement la concentration en butène-2 dans la coupe, ce qui favorise d'autant l'étape de métathèse et un rendement élevé en propylène.

L'objet principal de la première étape est de transformer le butadiène-1,3 et les n-butènes en butène-2. En effet, le butène-2 est la source du propylène qui est produit dans la dernière étape de métathèse en présence d'éthylène. Le butène-1 ne donne pas de produit nouveau avec l'éthylène, et il réagit avec le butène-2 pour donner du propylène, mais aussi des pentènes indésirables. Il est donc souhaitable de maximiser le rendement en butène-2, c'est-à-dire de se rapprocher autant que possible de la proportion autorisée par la thermodynamique. Le deuxième objet de cette étape est d'éliminer les traces d'hydrocarbures acétyléniques toujours présentes dans ces coupes et qui sont des poisons ou des polluants pour les étapes ultérieures.

Dans cette première étape (zone 1), on réalise donc simultanément les réactions suivantes, en présence d'hydrogène amené par un conduit 6 :
- hydroisomérisation sélective du butadiène-1,3 en un mélange de n-butènes à l'équilibre thermodynamique,
- isomérisation du butène-1 en butène-2, également à l'équilibre thermodynamique,
- hydrogénation des traces d'hydrocarbures acétyléniques.

Ces réactions peuvent être réalisées au moyen de divers catalyseurs spécifiques comprenant un ou plusieurs métaux, par exemple du groupe 10 de la classification périodique (Ni, Pd, Pt), déposés sur un support. On met en oeuvre de préférence un catalyseur qui comprend au moins un composé de palladium fixé sur un support minéral réfractaire, par exemple sur une alumine. La teneur en palladium sur le support peut être comprise entre 0,01 et 5 % en poids, de préférence entre 0,05 et 1 % en poids. Divers modes de prétraitement connus de l'homme de l'art peuvent éventuellement être appliqués à ces catalyseurs pour améliorer la sélectivité dans l'hydrogénation du butadiène-1,3 en butènes aux dépens de l'hydrogénation totale en butane qu'il faut éviter. Le catalyseur contient de préférence 0,05 à 10 % en poids de soufre. De façon avantageuse, on utilise un catalyseur constitué par du palladium déposé sur de l'alumine et qui contient du soufre.

La sulfuration du catalyseur peut être effectuée in situ (dans la zone de réaction) ou mieux ex situ. Dans ce dernier cas, on opère avantageusement selon le procédé décrit dans le brevet FR-93/09524, c'est-à-dire que le catalyseur a été traité, avant son chargement dans le réacteur d'hydrogénation, par au moins un composé soufré dilué dans un solvant, et que le catalyseur obtenu contenant 0,05 à 10 % de soufre (en poids) est chargé dans le réacteur et activé sous atmosphère neutre ou réductrice à une température comprise entre 20 et 300 °C, une pression comprise entre 0,1 et 5 MPa et une VVH comprise entre 50 et 600 h⁻¹, et que la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion de butadiène-1,3 dans la coupe est importante, ce qui est le cas par exemple d'une coupe de vapocraquage lorsqu'on ne souhaite pas en extraire le butadiène-1,3 pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie théorique.

Les conditions opératoires sont choisies de telle façon que les réactifs et les produits soient en phase liquide. Il peut cependant être avantageux de choisir un mode de fonctionnement tel que les produits soient partiellement vaporisés à la sortie du réacteur, ce qui facilite le contrôle thermique de la réaction. La température peut varier de 20 à 200 °C, de préférence de 50 à 150 °C ou mieux de 60 à 150 °C. La pression peut être ajustée entre 0,1 et 5 MPa, de préférence entre 0,5 et 4 MPa et avantageusement entre 0,5 et 3 MPa, de telle façon que les réactifs, au moins en partie, soient en phase liquide. La vitesse spatiale peut être comprise entre 0,5 et 10 h⁻¹ et de préférence entre 1 et 6 h⁻¹, avec un rapport H₂ / dioléfines (molaire) de 0,5 à 5 et de préférence de 1 à 3.

Le ou les réacteurs d'hydroisomérisation peuvent avantageusement être suivis d'une colonne de stabilisation qui élimine les traces d'hydrogène en excès et le méthane éventuel.

L'objet de la deuxième étape (zone 2) est de polymériser l'isobutène présent dans la coupe C₄ issue de l'étape précédente par exemple en polyisobutène pouvant entrer dans la composition d'élastomères, ou encore en polybutènes liquides utilisables comme additifs de lubrifiants.

L'effluent de l'étape 1 (zone 1) amené par une conduite 11 peut être traité tel quel (représentation de la figure 1).

Il peut être intéressant, pour améliorer la qualité des produits, d'extraire l'isobutène de l'effluent résultant de l'étape précédente avant polymérisation. Cette extraction peut se faire par toute méthode bien connue, par exemple simplement par distillation, ou bien par hydratation de l'isobutène en alcool butylique tertiaire en présence par exemple d'acide sulfurique, suivie d'une séparation et d'une déshydratation de l'alcool pour récupérer l'isobutène, ou bien encore par réaction avec du méthanol en présence d'une résine acide échangeuse d'ions pour transformer l'isobutène en méthyl-tertiobutyléther (MTBE) qui est facilement séparé par distillation puis décomposé en présence d'un catalyseur acide pour libérer l'isobutène purifié.

La figure 2 schématise cette possibilité avec une zone 4 de séparation de l'isobutène.

La polymérisation est réalisée au moyen d'un catalyseur acide de Lewis, par exemple du chlorure d'aluminium, un chloroalkylaluminium, du tétrachlorure d'étain, du trifluorure de bore, éventuellement associé à des traces d'acides de Bronsted comme l'acide chlorhydrique, l'eau, le chlorure de butyle tertiaire, les acides organiques. Le catalyseur peut être mis en oeuvre à l'état solide en poudre ou sous forme d'une suspension dans un hydrocarbure saturé tel que l'hexane ou l'isobutane, ou dans un hydrocarbure halogéné tel que le chlorure de méthyle.

Les conditions opératoires sont choisies de telle façon que la température de réaction puisse être contrôlée avec précision. On fixe en général la température pour que la réaction se déroule à une vitesse suffisante, par exemple de -100 à +100 °C, de préférence de -50 à +50 °C, et la pression est ajustée par exemple de telle façon que l'hydrocarbure éventuellement mis en oeuvre soit partiellement vaporisé par la chaleur dégagée par la polymérisation. Dans le cas ou le catalyseur est mis en oeuvre à l'état solide, la chaleur de réaction peut être enlevée par la circulation en boucle du mélange réactionnel à travers un échangeur extérieur.

La section réactionnelle est suivie d'une section dans laquelle le catalyseur est séparé de l'effluent, par exemple par un lavage avec de la soude suivi d'un lavage à l'eau, puis d'une section de séparation (par exemple de distillation) où on sépare le polymère sortant par un conduit 8.

Dans le cas où l'effluent total issu de l'étape 1 a été traité par l'étape 2, la distillation sépare un distillat comprenant la coupe C₄ résiduelle qui sort par le conduit 13 (figure 1).

Dans le cas où le fractionnement a été opéré avant polymérisation, un effluent comprenant la coupe C₄ résiduelle passe dans la conduite 13 (figure 2).

La coupe C₄ résiduelle ainsi obtenue contient en majorité des butanes et du butène-2. Dans la dernière étape 3 du procédé (zone 3), ce butène-2 est mis en réaction avec de l'éthylène (amené par la conduite 7) pour donner du propylène (sortant par la conduite 10) par métathèse. Un conduit 9 évacue les sous-produits séparés.

La réaction de métathèse de l'éthylène avec le butène-2 peut être catalysée par des oxydes métalliques variés déposés sur des supports. On utilise de préférence un catalyseur comportant au moins un oxyde de rhénium déposé sur un support composé par un oxyde réfractaire contenant au moins de l'alumine, qui présente un caractère acide, comme par exemple l'alumine elle-même, les silice-alumines ou les zéolithes.

On peut citer à titre d'exemple préféré les catalyseurs comportant de l'heptoxyde de rhénium déposé sur une alumine gamma analogue à celle utilisée dans les catalyseurs de reforming, comme décrits dans le brevet US 4 795 734. La teneur en rhénium (exprimée en rhénium métallique) peut être comprise entre 0,01 et 20 %, de préférence entre 1 et 15 % en poids. Les catalyseurs sont soumis par exemple à une activation thermique finale à une température comprise entre 400 et 1000 °C pendant une durée de 10 minutes à 5 heures sous une atmosphère non-réductrice.

Les catalyseurs comportant de l'heptoxyde de rhénium déposé sur une alumine peuvent aussi être modifiés par l'adjonction d'un oxyde d'un autre métal. De tels catalyseurs modifiés comprennent par exemple du rhénium à l'état d'oxyde, de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale, tel que décrit dans le brevet FR 2 709 125.

La réaction de métathèse est effectuée de préférence en phase liquide, en l'absence d'oxygène, de composés oxygénés et d'humidité, et à une température comprise entre 0 et 200 °C, de préférence entre 20 et 150 °C, sous une pression au moins égale à la tension de vapeur du mélange réactionnel à la température de réaction.

Le catalyseur peut être mis en oeuvre en lit fixe. Cependant, comme il doit être fréquemment régénéré, il est alors nécessaire de disposer d'au moins deux réacteurs en parallèle, l'un étant en fonctionnement pendant que l'autre est en régénération. On utilise de préférence un système de lit catalytique mobile comme il est décrit dans le brevet français FR 2 608 595.

Le catalyseur est soutiré à intervalles de temps réguliers en bas du réacteur et transféré vers un système de régénération en continu, d'où il est renvoyé en haut du réacteur.

Compte-tenu des limitations imposées par la thermodynamique, l'éthylène non converti est fractionné dans une première colonne de distillation et recyclé au réacteur de métathèse. Une deuxième colonne de distillation sépare le propylène, et une troisième colonne sépare les hydrocarbures en C₄ non convertis qui peuvent être recyclés au réacteur de métathèse, et une fraction plus lourde en faible quantité.

A la coupe C₄ traitée dans le procédé selon l'invention, on peut très avantageusement adjoindre un effluent d'hydrocarbures en C₄, essentiellement du butène-1 et de l'isobutène, provenant du traitement d'une coupe C₅ par hydroisomérisation puis métathèse avec de l'éthylène, selon le schéma de la figure 3.

La figure 3 illustre ce mode de réalisation, dans lequel la coupe C₄ et une coupe C₅ sont traitées simultanément et séparément.

La coupe C₅ contient principalement des pentanes, des méthyl-butènes, des pentènes et des dioléfines à 5 atomes de carbone, accompagnés de faibles quantités d'hydrocarbures acétyléniques.

La coupe C₅ entrant par la canalisation 20 est soumise à une étape d'hydroisomérisation (dans les conditions décrites ci-dessus pour la coupe C₄) dans une zone 21 avec introduction d'hydrogène par une conduite 22. L'effluent sortant par la canalisation 23 est au moins en partie soumis à une étape de métathèse dans une zone 24 avec de l'éthylène amené par un conduit 25. Il est séparé : du propylène sortant par une conduite 26, des sous-produits sortant par une conduite 27, et un mélange butène-1 et isobutène sortant par une conduite 28.

Par la conduite 28, le mélange butène-1 et isobutène est amené dans la zone 1 d'hydroisomérisation de la coupe C₄. La coupe C₄ et ledit mélange sont alors traités selon le procédé décrit précédemment.

D'une façon encore plus simplifiée, il peut être avantageux de mélanger l'effluent de l'étape 2 (zone 2) de polymérisation de l'isobutène sur la chaîne C₄, et qui sort par la conduite 13, avec l'effluent de l'étape 1 (zone 21) d'hydroisomérisation de la coupe C₅ (amenée par la conduite 20) qui sort par la canalisation 23, de manière à ne conserver qu'une seule opération de métathèse (zone 3) comme indiqué dans la figure 4.

Lorsque le procédé est appliqué à une coupe C₄ de vapocraquage, il peut être avantageux d'intégrer l'unité de métathèse avec le craqueur, de manière à bénéficier du train de fractionnement de celui-ci.

L'invention concerne également une installation pour mettre en oeuvre le procédé décrit ci-dessus et qui comporte successivement :
1) une zone 1 d'hydrogénation sélective avec isomérisation simultanée du butène-1 en butènes-2, ladite zone comportant au moins un moyen 5 pour l'introduction de la coupe à convertir, au moins un moyen 11 pour la sortie de l'effluent, au moins un moyen 6 pour l'introduction de l'hydrogène, ladite zone comportant également au moins un lit de catalyseur, qui comprend de préférence au moins un métal choisi dans le groupe formé par le nickel, le palladium et le platine, déposé sur un support,
2) une zone 2 de polymérisation et de séparation, comportant au moins un moyen 11, 12 pour l'introduction de l'isobutène de l'effluent issu de la zone 1, au moins un moyen 8 pour la sortie du polymère, au moins un moyen 13 pour la sortie de la coupe C₄ résiduelle sans polymère, la zone de polymérisation contenant au moins un catalyseur acide,
3) une zone 3 de métathèse contenant au moins un catalyseur de préférence à base d'oxyde de rhénium déposé sur un support, suivie d'une zone de séparation, et comprenant au moins un moyen d'introduction 13 de la coupe C₄ résiduelle, au moins un moyen 7 d'introduction de l'éthylène, au moins un moyen 10 pour la sortie du propylène et au moins un moyen 9 pour la sortie de sous-produits.

Dans une première réalisation, la zone 2 de l'étape 2 comprend une zone de polymérisation suivie d'au moins une zone de séparation. Avantageusement, la zone de polymérisation contient un catalyseur acide en suspension.

Dans une réalisation préférée, la zone 2 de l'étape 2 est précédée d'une zone 4 d'extraction de l'isobutène et comprend une zone de polymérisation suivie d'au moins une zone de séparation.

On opère de préférence avec dans la zone 3 de l'étape 3 un lit mobile de catalyseur.

L'installation pour mettre en oeuvre le procédé décrit ci-dessus peut comporter aussi une section de traitement d'une coupe C₅ oléfinique pour produire une partie au moins de la coupe C₄ à convertir, ladite coupe C₅ pouvant contenir notamment des dioléfines, des n-pentènes, des méthylbutènes et des impuretés acétylèniques, qui comprend successivement au moins :
1) une zone 21 d'hydrogénation sélective avec isomérisation simultanée des oléfines alpha à 5 atomes de carbone en pentènes-2 et en méthyl-2 butène-2, ladite zone comportant au moins un moyen 20 pour l'introduction de la coupe C₅, au moins un moyen 23 pour la sortie de l'effluent, au moins un moyen 22 pour l'introduction de l'hydrogène, ladite zone comportant également au moins un lit de catalyseur qui comprend de préférence au moins un métal choisi dans le groupe formé par le nickel, le palladium et le platine, déposé sur un support,
2) une zone 24 de métathèse contenant au moins un catalyseur de préférence à base d'oxyde de rhénium déposé sur un support, et comprenant au moins un moyen 23 d'introduction de l'effluent issu de la zone 21, au moins un moyen 25 d'introduction de l'éthylène, au moins un moyen 26 pour la sortie du propylène, au moins un moyen 27 pour la sortie de sous-produits et au moins un moyen 28 pour la sortie d'un mélange de butène-1 et d'isobutène, et qui est envoyé dans la zone 1.

De façon particulièrement intéressante, les coupes C₄ et/ou C₅ proviennent d'une zone de vapocraquage en amont, le (les) moyen (s) d'introduction des coupes étant relié (s) à ladite zone de vapocraquage, et le moyen d'introduction de l'éthylène dans l'étape de métathèse étant relié à ladite zone de vapocraquage.

Les exemples suivants illustrent l'invention sans en limiter la portée.

### EXEMPLE 1

Une coupe C₄ en sortie de vapocraqueur présente la composition indiquée dans le tableau 1 (flux 1). Abréviations du tableau : MAPD = méthyl-acétylène + propadiène, BBV = butadiène-1,2 + butyne-1 + vinyl-acétylène.

Cette coupe C₄ est d'abord soumise à une hydroisomérisation. Elle est introduite en continu, avec le débit massique indiqué dans le tableau 1, et sous une pression de 2 MPa, dans un premier réacteur comprenant un lit fixe de 2,6 T d'un catalyseur constitué par du palladium sur alumine sulfuré au préalable. De l'hydrogène (mélangé à du méthane) est également injecté dans ce réacteur, comme indiqué dans le tableau 1 (flux 2). La température, qui est de 50 °C à l'entrée, s'élève à 95 °C à la sortie. L'effluent est ensuite traité dans un réacteur de finition chargé avec 2,5 T du même catalyseur, et fonctionnant pratiquement en isotherme à 70 °C. A la sortie (tableau 1, flux 3), la coupe est débarrassée des composés acétyléniques, et le butadiène-1,3 a été transformé essentiellement en butènes, qui sont en majorité des butènes-2, le butène-1 ayant été isomérisé. La coupe est alors traitée dans une colonne de stabilisation, où l'hydrogène résiduel et le méthane sont séparés. Après ce traitement, la coupe a la composition du flux 4 (tableau 1).

Dans la deuxième étape, l'isobutène contenu dans la coupe hydrotraitée est polymérisé pour donner un polymère constitué principalement d'unités isobutène mais contenant aussi en petites quantités des unités butène-1 (polybutène). Le flux 4 (tableau 1) est introduit dans un réacteur agité en même temps que le catalyseur constitué par du chlorure d'aluminium en poudre, à raison de 6 kg/h d'AlCl₃. La chaleur de polymérisation est éliminée en faisant circuler en boucle la phase liquide du réacteur à travers un échangeur extérieur réfrigéré de manière à maintenir la température de réaction à -5 °C. A la sortie du réacteur, le catalyseur est détruit et séparé de l'effluent brut par un lavage à la soude caustique suivi d'un lavage à l'eau. L'effluent brut débarrassé du catalyseur a la composition donnée dans le tableau 1 (flux 5). La coupe C₄ résiduelle et le polybutène sont séparés par distillation.

Dans la dernière étape, la coupe résiduelle qui contient principalement des butènes-2 est mise en réaction avec de l'éthylène (composition globale : flux 6 du tableau 1) sur un catalyseur de métathèse, constitué par de l'oxyde de rhénium sur alumine gamma (8 % en poids en rhénium métal), préparé selon les enseignements du brevet US 4 795 734. La coupe C₄ est mélangée à l'entrée du réacteur de métathèse avec de l'éthylène d'appoint, ainsi qu'avec des flux de recyclage d'éthylène et de butènes. Ce réacteur fonctionne en lit mobile, comme décrit dans le brevet FR 2 608 595, à une température de 35 °C et sous une pression de 3,5 MPa, et il est couplé avec un régénérateur fonctionnant à 550 °C sous pression atmosphérique. Le catalyseur est soutiré à intervalles de temps réguliers en bas du réacteur et transféré vers le régénérateur, d'où il est renvoyé en haut du réacteur, les transferts se faisant à travers des sas tampons. En sortie de réacteur, l'éthylène non converti est fractionné dans une première colonne de distillation et recyclé. Une deuxième colonne de distillation sépare le propylène, et une troisième colonne sépare les hydrocarbures en C₄ non convertis qui sont également recyclés, et des pentènes en faible quantité. La composition de l'effluent de métathèse est indiquée dans le tableau 1, flux 7.

Le bilan global de la transformation s'établit donc comme suit. Pour 100 parties en poids (pp) de coupe C₄ sortie du vapocraqueur, on consomme 1,6 pp d'hydrogène et 26,7 pp d'éthylène, et on produit 29,7 pp de polybutène et 79,6 pp de propylène. Au niveau du vapocraqueur d'où est issue la coupe C₄ traitée, ce bilan représente donc une faible consommation d'éthylène, qui permet une production supplémentaire de propylène importante, et ceci sans avoir à modifier les conditions de marche du craqueur.

### EXEMPLE 2

Cet exemple décrit la mise en oeuvre à la fois d'une coupe C₄ et d'une coupe C₅ de vapocraquage pour produire du polybutène et du propylène.

Une coupe C₅ en sortie de vapocraqueur présente la composition indiquée dans le tableau 2 (flux 1). Cette coupe C₅ est soumise d'abord à une étape d'hydroisomérisation, puis à une étape de métathèse en présence d'éthylène, pour produire du propylène et une fraction C₄ constituée essentiellement par de l'isobutène et du butène-1, qui sera mélangée avec la coupe C₄ issue du craqueur.

Dans la première étape d'hydroisomérisation, la coupe C₅ est introduite en continu, avec le débit massique indiqué dans le tableau 2, et sous une pression de 2 MPa, dans un premier réacteur comprenant un lit fixe de 1,2 T d'un catalyseur constitué par du palladium sur alumine sulfuré au préalable. De l'hydrogène (mélangé à du méthane) est également injecté dans ce réacteur, comme indiqué dans le tableau 2 (flux 2). La température, qui est de 50 °C à l'entrée, s'élève à 85 °C à la sortie. L'effluent est ensuite traité dans un réacteur de finition chargé avec 1,1 T du même catalyseur, et fonctionnant pratiquement en isotherme à 80 °C. A la sortie (tableau 2, flux 3), la coupe est débarrassée des traces de composés acétyléniques, et les dioléfines en C₅ ont été hydrogénées en méthyl-butènes et en pentènes, qui sont en majorité du méthyl-2 butène-2 et du pentène-2 en raison de l'isomérisation qui se fait en même temps. La coupe est alors traitée dans une colonne de stabilisation, où l'hydrogène résiduel et le méthane sont séparés. Après ce traitement, la coupe a la composition du flux 4 (tableau 2).

Dans la deuxième étape, la coupe C₅ est mise en réaction avec de l'éthylène (composition globale : flux 5 du tableau 2) sur un catalyseur de métathèse, constitué par de l'oxyde de rhénium sur alumine gamma (8 % en poids en rhénium métal), préparé selon les enseignements du brevet US 4 795 734. Le réacteur de métathèse fonctionne en lit mobile, comme décrit dans le brevet FR 2 608 595, à une température de 35 °C et sous une pression de 3,5 MPa, et il est couplé avec un régénérateur fonctionnant à 550 °C sous pression atmosphérique. Le catalyseur est soutiré à intervalles de temps réguliers en bas du réacteur et transféré vers le régénérateur, d'où il est renvoyé en haut du réacteur, les transferts se faisant à travers des sas tampons. En sortie de réacteur, l'éthylène non converti est fractionné dans une première colonne de distillation et recyclé. Une deuxième colonne de distillation sépare le propylène, et une troisième colonne sépare l'isobutène et le butène-1 en tête, et des oléfines plus lourdes en fond. La composition de l'effluent de métathèse est indiquée dans le tableau 2, flux 6.

Le flux d'isobutène et de butène-1 obtenu après fractionnement de l'effluent brut de métathèse (tableau 3, flux 2) est alors mélangé avec la coupe C₄ mise en oeuvre dans l'exemple 1 (tableau 3, flux 1). Le mélange est soumis à la même succession d'étapes et dans les mêmes conditions que décrit dans l'exemple 1. Le bilan de l'opération est donné dans le tableau 3.

Le bilan global de la transformation s'établit donc comme suit. Pour 69 parties en poids (pp) de coupe C₄ et 31 parties en poids (pp) de coupe C₅ sortie du vapocraqueur, on consomme 1,5 pp d'hydrogène et 23,4 pp d'éthylène, et on produit 24,6 pp de polybutène et 62,7 pp de propylène.

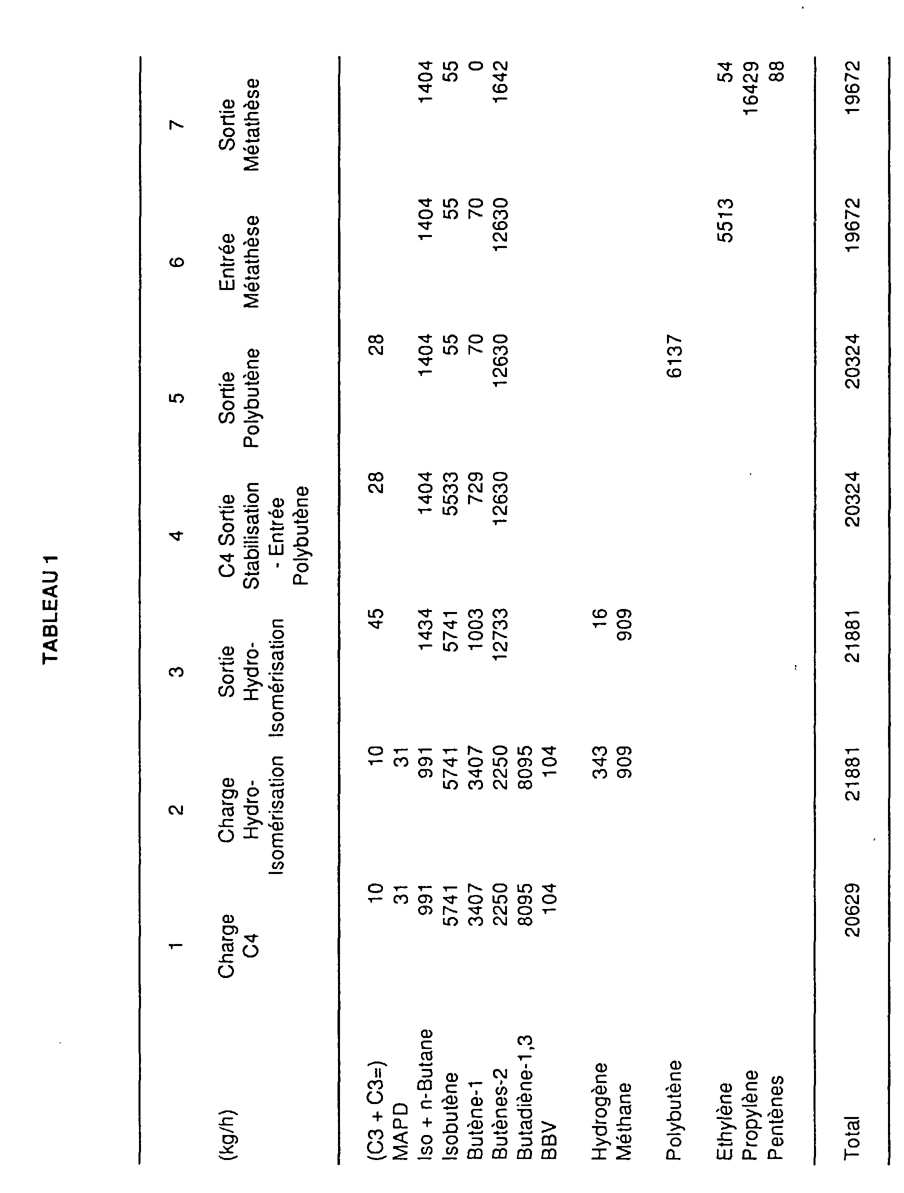

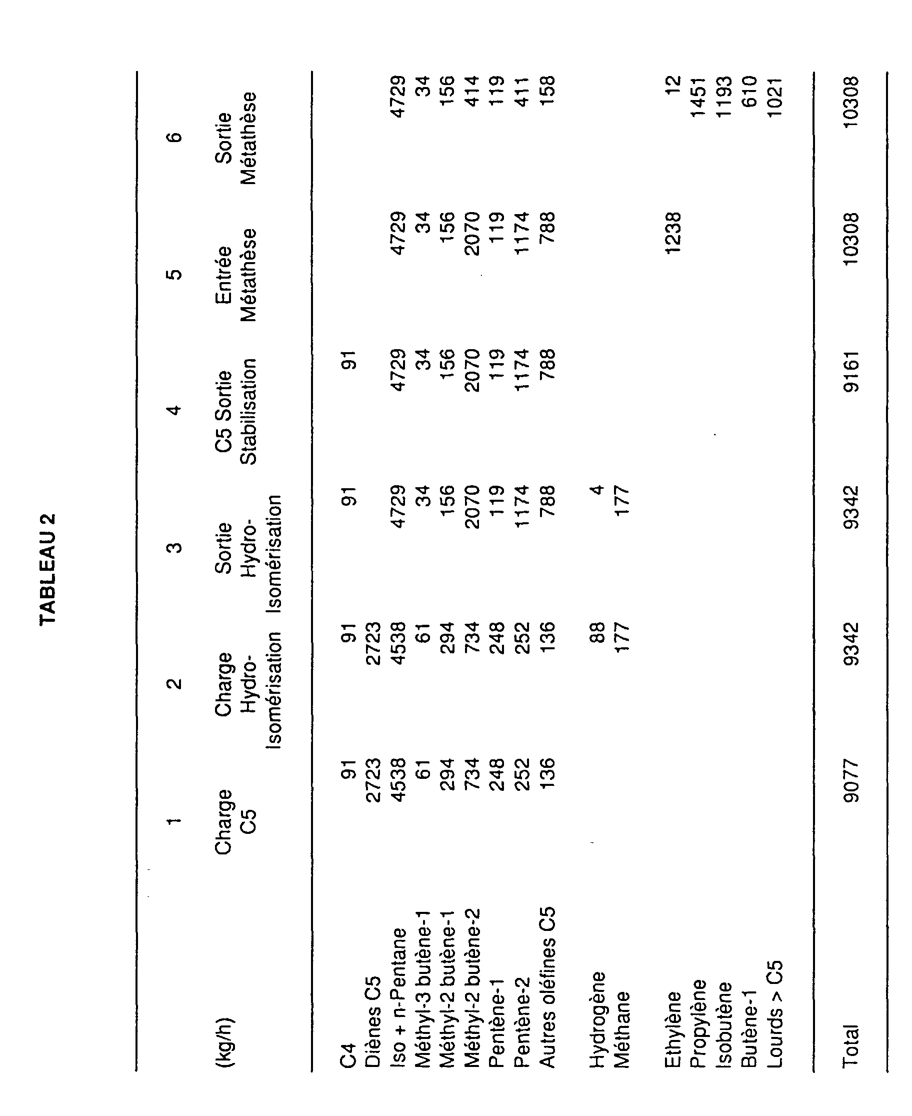

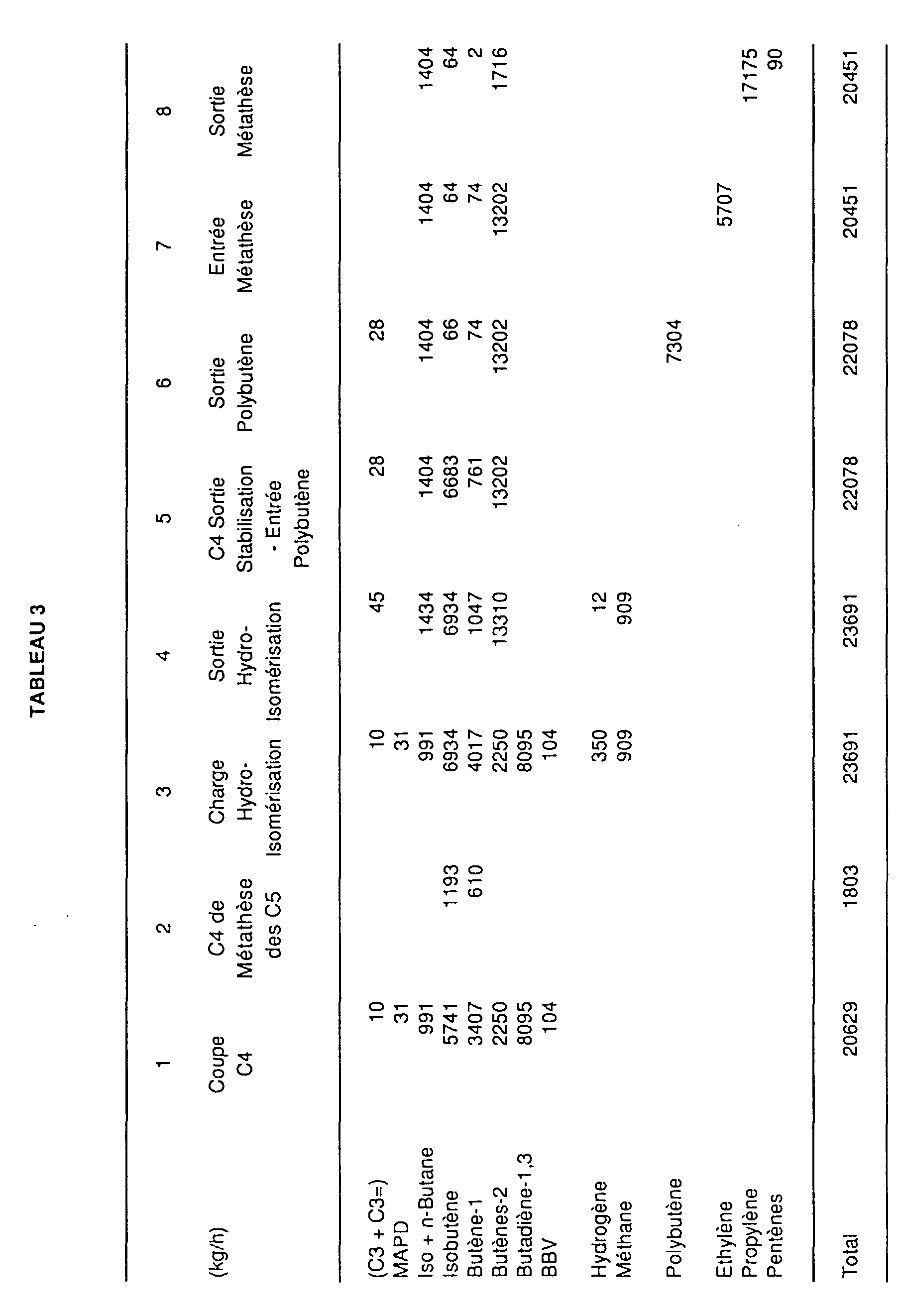

## Revendications

1. Procédé de conversion d'une coupe C₄ oléfinique en polyisobutène et en propylène, ladite coupe contenant notamment des dioléfines, du butène-1, de l'isobutène et des impuretés acétylèniques, caractérisé en ce que ledit procédé comporte les étapes suivantes se déroulant successivement :
1) hydrogénation sélective des dioléfines et des impuretés acétyléniques avec simultanément isomérisation du butène-1 en butènes-2, par passage de ladite coupe en phase liquide sur un catalyseur comprenant au moins un métal choisi dans le groupe formé par le nickel, le palladium et le platine, déposé sur un support, à une température de 20-200 °C, une pression de 1-5 MPa, une vitesse spatiale de 0,5-10 h⁻¹, avec un rapport H₂ / dioléfines (molaire) de 0,5 à 5, de façon à obtenir un effluent contenant majoritairement du butène-2 et de l'isobutène, et ne contenant pratiquement pas de dioléfines ni de composés acétyléniques,
2) polymérisation de l'isobutène contenu dans l'effluent de l'étape 1 en polyisobutène, en présence d'un catalyseur acide, à une température de -100 à +100 °C, à une pression telle que la réaction se déroule en phase liquide, une séparation de l'isobutène des autres constituants de la coupe issue de l'étape 1 pouvant avoir lieu avant la polymérisation, pour obtenir d'une part du polyisobutène et d'autre part une coupe C₄ résiduelle sans polymère,
3) métathèse de la coupe C₄ résiduelle avec de l'éthylène, en présence d'un catalyseur comportant au moins un oxyde de rhénium déposé sur un support, à une température comprise entre 0 et 100 °C, et une pression au moins égale à la tension de vapeur du mélange réactionnel à la température de réaction, de façon à obtenir un effluent contenant du propylène, la métathèse étant suivie d'une séparation du propylène.

2. Procédé selon la revendication 1, caractérisé en ce que l'étape 1) se déroule en présence d'un catalyseur contenant 0,05 à 10 % en poids de soufre.

3. Procédé selon l'une des revendications précédentes, caractérisé en ce que le catalyseur a été traité, avant son chargement dans le réacteur d'hydrogénation, par au moins un composé soufré dilué dans un solvant, et que le catalyseur obtenu contenant 0,05 à 10 % de soufre (en poids) est chargé dans le réacteur et activé sous atmosphère neutre ou réductrice à une température comprise entre 20 et 300 °C, une pression comprise entre 0,1 et 5 MPa et une VVH comprise entre 50 et 600 h⁻¹, et que la charge est mise au contact dudit catalyseur activé.

4. Procédé selon l'une des revendications précédentes, caractérisé en ce que le catalyseur est constitué par du palladium déposé sur de l'alumine et qui contient du soufre.

5. Procédé selon l'une des revendications précédentes, caractérisé en ce que la polymérisation de l'isobutène se déroule en présence d'un catalyseur choisi dans le groupe formé par le chlorure d'aluminium, le trifluorure de bore, les chloroalkylaluminium, le tétrachlorure d'étain.

6. Procédé selon l'une des revendications précédentes, caractérisé en ce que la polymérisation de l'isobutène se déroule en présence d'un catalyseur acide associé à des traces d'acide de Bronsted.

7. Procédé selon l'une des revendications précédentes, caractérisé en ce que la métathèse a lieu en présence d'un catalyseur contenant de l'oxyde de rhénium à raison de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale.

8. Procédé selon l'une des revendications précédentes, caractérisé en ce que la métathèse est effectuée avec un catalyseur en lit mobile.

9. Procédé selon la revendication 1, caractérisé en ce que, dans la coupe C₄, l'isobutène et le butène-1 peuvent être issus au moins en partie d'une coupe C₅ oléfinique qui est soumise à un traitement comportant au moins les deux étapes suivantes se déroulant successivement :
1) hydrogénation sélective des dioléfines et des impuretés acétyléniques avec simultanément isomérisation des oléfines alpha en C₅ en pentènes-2 et en méthyl-2 butène-2, par passage de ladite coupe C₅ en phase liquide sur un catalyseur comprenant au moins un métal choisi dans le groupe formé par le nickel, le palladium et le platine, déposé sur un support, à une température de 20-200 °C, une pression de 1-5 MPa, une vitesse spatiale de 0,5-10 h⁻¹, avec un rapport H₂/dioléfines (molaire) de 0,5 à 5, de façon à obtenir un effluent contenant majoritairement du pentène-2 et du méthyl-2 butène-2, et ne contenant pratiquement pas de dioléfines ni de composés acétyléniques,
2) métathèse d'au moins une partie de l'effluent issu de l'étape précédente avec de l'éthylène, en présence d'un catalyseur comportant au moins un oxyde de rhénium déposé sur un support, à une température comprise entre 0 et 100 °C, et une pression au moins égale à la tension de vapeur du mélange réactionnel à la température de réaction, de façon à obtenir un effluent contenant principalement du propylène, du butène-1 et de l'isobutène, la métathèse étant suivie d'une séparation du propylène,
3) introduction du mélange butène-1 et isobutène dans l'étape d'hydrogénation de la coupe C₄.

10. Procédé selon l'une des revendications précédentes, caractérisé en ce que les coupes C₄ et C₅ sont issues d'une unité de vapocraquage.

11. Procédé selon la revendication 9, caractérisé en ce que l'étape unique de métathèse traite simultanément l'effluent d'hydroisomérisation de la coupe C₅ et la coupe C₄ résiduelle.

12. Installation pour la conversion d'une coupe C₄ oléfinique en polyisobutène et en propylène, caractérisée en ce qu'elle comporte successivement :
1) une zone 1 d'hydrogénation sélective avec isomérisation simultanée du butène-1 en butènes-2, ladite zone comportant au moins un moyen 5 pour l'introduction de la coupe à convertir, au moins un moyen 11 pour la sortie de l'effluent, au moins un moyen 6 pour l'introduction de l'hydrogène, ladite zone comportant également au moins un lit de catalyseur,
2) une zone 2 de polymérisation et de séparation, comportant au moins un moyen pour l'introduction de l'isobutène de l'effluent issu de la zone 1, au moins un moyen 8 pour la sortie du polymère, au moins un moyen 13 pour la sortie de la coupe C₄ résiduelle sans polymère, la zone de polymérisation contenant au moins un catalyseur acide,
3) une zone 3 de métathèse contenant au moins un catalyseur suivie d'une zone de séparation, et comprenant au moins un moyen 13 d'introduction de la coupe C₄ résiduelle, au moins un moyen 7 d'introduction de l'éthylène, au moins un moyen 10 pour la sortie du propylène et au moins un moyen 9 pour la sortie de sous-produits.

13. Installation selon la revendication 12, caractérisée en ce que la zone 2 est précédée d'une zone d'extraction de l'isobutène, et qu'elle comprend une zone de polymérisation suivie d'au moins une zone de séparation.

14. Installation selon l'une des revendications 12 ou 13, caractérisée en ce que la zone 2 comprend une zone de polymérisation suivie d'au moins une zone de séparation.

15. Installation selon la revendication 11, caractérisée en ce que la zone 3 contient un lit mobile de catalyseur.

16. Installation selon l'une des revendications précédentes, caractérisée en ce qu'elle comporte aussi une section de traitement d'une coupe C₅ oléfinique pour produire une partie au moins de la coupe C₄ à convertir, ladite coupe C₅ pouvant contenir notamment des dioléfines, des n-pentènes, des méthylbutènes et des impuretés acétylèniques, qui comprend successivement au moins :
1) une zone 21 d'hydrogénation sélective avec isomérisation simultanée des oléfines alpha à 5 atomes de carbone en pentènes-2 et en méthyl-2 butène-2, ladite zone comportant au moins un moyen 20 pour l'introduction de la coupe C₅, au moins un moyen 23 pour la sortie de l'effluent, au moins un moyen 22 pour l'introduction de l'hydrogène, ladite zone comportant également au moins un lit de catalyseur,
2) une zone 24 de métathèse comprenant au moins un moyen 23 d'introduction de l'effluent issu de la zone 21, au moins un moyen 25 d'introduction de l'éthylène, au moins un moyen 26 pour la sortie du propylène,au moins un moyen 27 pour la sortie de sous-produits et au moins un moyen 28 pour la sortie d'un mélange de butène-1 et d'isobutène, et qui est envoyé dans la zone 1.

17. Installation selon l'une des revendications 12 à 16, caractérisée en ce que le (les) moyen (s) d'introduction de la coupe à convertir est relié à une zone de vapocraquage produisant une coupe C₄ ou C₅, et en ce que le moyen d'introduction de l'éthylène dans la zone de métathèse est relié à ladite zone de vapocraquage.

## Claims

1. A process for the conversion of an olefinic C₄ cut to polyisobutene and to propylene, said cut containing mainly diolefins, 1-butene, isobutene and acetylenic impurities, characterized in that said process comprises the following steps which are carried out successively:
1) selective hydrogenation of the diolefins and acetlyenic impurities with simultaneous isomerisation of the 1-butene to 2-butenes, by passing said cut in the liquid phase over a catalyst comprising at least one metal selected from the group formed by nickel, palladium and platinum, deposited on a support, at a temperature of 20-200°C, a pressure of 1-5 Mpa, and a space velocity of 0.5-10 h⁻¹, with a H₂/diolefin (molar) ratio of 0.5 to 5, to obtain an effluent containing mainly 2-butene and isobutene, and containing practically no diolefins or acetylenic compounds;
2) polymerisation of the isobutene contained in the effluent from step 1 to polyisobutene, in the presence of an acid catalyst, at a temperature of -100°C to +100°C, and at a pressure such that the reaction is carried out in the liquid phase, wherein the isobutene may be separated from the other constituents of the cut from step 1 before polymerisation, to obtain polyisobutene and a residual C₄ cut without a polymer;
3) metathesis of the residual C₄ cut with ethylene, in the presence of a catalyst comprising at least one rhenium oxide deposited on a support, at a temperature in the range 0°C to 100°C, and at a pressure which is at least equal to the vapour tension of the reaction mixture at the reaction temperature, to obtain an effluent containing propylene, metathesis being followed by separation of the propylene.

2. A process according to claim 1, characterized in that step 1) is carried out in the presence of a catalyst containing 0.05% to 10% by weight of sulphur.

3. A process according to claim 1 or claim 2, characterized in that the catalyst has been treated, before charging it into the hydrogenation reactor, with at least one sulphur-containing compound diluted in a solvent, and that the catalyst obtained containing 0.05% to 10% (by weight) of sulphur is charged into the reactor and activated in a neutral or reducing atmosphere at a temperature in the range 20°C to 300°C, a pressure in the range 0.1 to 5 MPa and a GHSV in the range 50 to 600 h⁻¹, and in that the feed is brought into contact with said activated catalyst.

4. A process according to any one of the preceding claims, characterized in that the catalyst is constituted by palladium deposited on alumina and containing sulphur.

5. A process according to any one of the preceding claims, characterized. in that the isobutene is polymerised in the presence of a catalyst selected from the group formed by aluminium chloride, boron trifluoride, chloroalkylaluminium compounds and tin tetrachloride.

6. A process according to any one of the preceding claims, characterized in that the isobutene is polymerised in the presence of an acid catalyst associated with traces of a Bronsted acid.

7. A process according to any one of the preceding claims, characterized in that metathesis takes place in the presence of a catalyst containing a rhenium oxide in a proportion of 0.01% to 20% by weight , expressed as rhenium metal, deposited on a support containing at least 75% by weight of alumina and 0.01% to 30% by weight of at least one oxide of a metal selected from the group formed by niobium and tantalum.

8. A process according to any one of the preceding claims, characterized in that metathesis is carried out with a catalyst in a mobile bed.

9. A process according to claim 1, characterized in that in the C₄ cut, the isobutene and 1-butene originate at least in part from an olefinic C5 cut which is treated in at least the following two steps which are carried out successively:
1) selective hydrogenation of the diolefins and acetylenic impurities with simultaneous isomerisation of the C₅ alpha olefins to 2-pentenes and 2-methyl-2-butene, by passing said C₅ cut in the liquid phase over a catalyst comprising at least one metal selected from the group formed by nickel, palladium and platinum, deposited on a support, at a temperature of 20-200°C, a pressure of 1-5 MPa, and a space velocity of 0.5-10 h⁻¹, with a H₂/diolefin (molar) ratio of 0.5 to 5, to obtain an effluent containing mainly 2-pentene and 2-methyl-2-butene, and containing practically no diolefins or acetylenic compounds;
2) metathesis of at least a portion of the effluent from the preceding step with ethylene, in the presence of a catalyst comprising at least one rhenium oxide deposited on a support, at a temperature in the range 0°C to 100°C, and at a pressure which is at least equal to the vapour tension of the reaction mixture at the reaction temperature, to obtain an effluent containing mainly propylene, 1-butene and isobutene, metathesis being followed by separation of the propylene;
3) introduction of the 1-butene and isobutene mixture to the selective hydrogenation step for the C₄ cut.

10. A process according to any one of the preceding claims, characterized in that the C₄ and C₅ cuts originate from a steam cracking unit.

11. A process according to claim 9, characterized in that the single metathesis step simultaneously treats the hydroisomerisation effluent from the C₅ cut and the residual C₄ cut.

12. A plant for converting an olefinic C₄ cut to polyisobutene and to propylene, characterized in that it comprises successively:
1) a selective hydrogenation zone 1 with simultaneous isomerisation of 1-butene to 2-butenes, said zone comprising at least one means 5 for introducing a cut to be converted, at least one means 11 for discharging an effluent, and at least one means 6 for introducing hydrogen, said zone also comprising at least one catalyst bed;
2) a polymerisation and separation zone 2 comprising at least one means for introducing isobutene from the effluent from zone 1, at least one means 8 for discharging polymer, and at least one means 13 for discharging a residual C₄ cut without polymer, the polymerisation zone containing at least one acid catalyst;
3) a metathesis zone 3 containing at least one catalyst, followed by a separation zone, and comprising at least one means 13 for introducing a residual C₄ cut, at least one means 7 for introducing ethylene, at least one means 10 for discharging propylene and at least one means 9 for discharging by-products.

13. A plant according to claim 12, characterized in that zone 2 is preceded by an isobutene extraction zone, and in that it comprises a polymerisation zone followed by at least one separation zone.

14. A plant according to claim 12 or claim 13, characterized in that zone 2 comprises a polymerisation zone followed by at least one separation zone.

15. A plant according to claim 11, characterized in that zone 3 contains a mobile catalyst bed.

16. A plant according to any one of the preceding claims, characterized in that it further comprises a section for treating an olefinic C₅ cut to produce at least a portion of the C₄ cut to be converted, said C₅ cut containing diolefins, n-pentenes, methylbutenes and acetylenic impurities in particular, the plant comprising successively at least:
1) a selective hydrogenation zone 21 with simultaneous isomerisation of alpha olefins containing 5 carbon atoms to 2-pentenes and 2-methyl-2-butene, said zone comprising at least one means 20 for introducing C₅ cut, at least one means 23 for discharging effluent, and at least one means 22 for introducing hydrogen, said zone also comprising at least one bed of a catalyst;
2) a metathesis zone 24 containing at least one means 23 for introducing effluent from zone 21, at least one means 25 for introducing ethylene, at least one means 26 for discharging propylene, at least one means 27 for discharging by-products and at least one means 28 for discharging a mixture of 1-butene and isobutene to leave, which is sent to zone 1.

17. A plant according to any one of claims 12 to 16, characterized in that the means for introducing the cut to be converted is/are connected to a steam cracking zone producing a C₄ or C₅ cut, and in that the means for introducing ethylene into the metathesis zone is connected to said steam cracking zone.

## Patentansprüche

1. Verfahren zur Umwandlung eines olefinischen C₄-Schnittes in Polyisobuten und Ployisopropylen, wobei dieser Schnitt insbesondere Diolefine, 1-Buten, Isobuten und acetylenische Verunreinigungen enthält, dadurch gekennzeichnet, dass dieses Verfahren die folgenden nacheinander ablaufenden Stufen aufweist:
1) selektive Hydrierung der Diolefine und der acetylenischen Verunreinigungen mit gleichzeitiger Isomerisierung von 1-Buten in 2-Butene durch Passieren dieses Schnittes in flüssiger Phase über einen Katalysator, der wenigstens ein aus der durch Nickel, Palladium und Platin gebildeten Gruppe gewähltes Metall umfasst, das auf einem Träger bei einer Temperatur von 20 - 200°C, einem Druck von 1 - 5 MPa, einer Raumgeschwindigkeit von 0,5 - 10 h⁻¹ abgeschieden ist, in einer Weise, um einen Abstrom zu erhalten, der hauptsächlich 2-Butene und Isobuten enthält und praktisch weder Diolefine noch acetylenische Verbindungen enthält,
2) Polymerisation des in dem Abstrom der Stufe 1 enthaltenen Isobutens zu Polyisobuten in Gegenwart eines sauren Katalysators bei einer Temperatur von-100 bis +100°C, bei einem Druck derart, dass die Reaktion in flüssiger Phase abläuft, wobei eine Trennung des Isobutens von den anderen Bestandteilen des Schnittes aus Stufe 1 vor der Polymerisation stattfinden kann, um einerseits Polyisobuten und andererseits einen restlichen C4-Schnitt ohne Polymer zu erhalten,
3) Metathesis bzw. Umsetzung des restlichen C₄-Schnittes mit Ethylen in Gegenwart eines Katalysators, der wenigstens ein auf einem Träger abgeschiedenes Oxid von Rhenium umfasst bei einer Temperatur, die zwischen 0 und 100°C liegt und einem Druck, der wenigstens gleich der Dampfspannung der Reaktionsmischung bei der Reaktionstemperatur ist, in einer Weise, um einen Abstrom zu erhalten, der Propylen enthält, wobei sich an die Metathesis bzw. Umsetzung eine Trennung des Propylens anschließt.

2. Verfahren nach Anspruch 1, dadurch gekennzeichnet, dass die Stufe 1) in Gegenwart eines Katalysators abläuft, der 0,05 bis 10 Gew.-% Schwefel enthält.

3. Verfahren nach einem der vorhergehenden Ansprüche, **dadurch gekennzeichnet**, dass der Katalysator vor seiner Beschickung in den Hydrierungsreaktor mit wenigstens einer in einem Lösungsmittel verdünnten schwefelhaltigen Verbindung behandelt worden ist und dass der erhaltene, 0,05 bis 10% (Gew.-%) Schwefel umfassende Katalysator in den Reaktor geladen und unter neutraler oder reduzierender Atmosphäre bei einer Temperatur, die zwischen 20 und 300°C liegt, einem Druck, der zwischen 0,1 und 5 MPa liegt, und einer VVH, die zwischen 50 und 600h⁻¹ liegt, aktiviert wird und dass die Charge in Kontakt mit diesem aktivierten Katalysator gebracht wird.

4. Verfahren nach einem der vorhergehenden Ansprüche, **dadurch gekennzeichnet**, dass sich der Katalysator durch auf Aluminiumoxid abgeschiedenes Palladium zusammensetzt und Schwefel umfasst.

5. Verfahren nach einem der vorhergehenden Ansprüche, **dadurch gekennzeichnet,** dass die Polymerisation des Isobutens in Gegenwart eines Katalysators abläuft, der in der durch Aluminiumchlorid, Bortrifuorid, den Aluminiumchloralkylen, Zinntetrachlorid gebildeten Gruppe gewählt ist.

6. Verfahren nach einem der vorhergehenden Ansprüche, **dadurch gekennzeichnet**, dass die Polymerisation des Isobutens in Gegenwart eines sauren, mit Spuren von Brönstedsäure verbunden Katalysators abläuft.

7. Verfahren nach einem der vorhergehenden Ansprüche, **dadurch gekennzeichnet**, dass die Metathesis bzw. Umsetzung in Gegenwart eines Katalysators stattfindet, der Rheniumoxid zu einem Anteil von 0,01 bis 20 Gew.-% ausgedrückt als metallisches Rhenium abgeschieden auf einen Träger umfasst, der wenigstens 75 Gew.-% Aluminiumoxid und 0,01 bis 30 Gew.-% wenigstens eines Oxids eines Metalls enthält, das aus der durch Niob und Tantal gebildeten Gruppe gewählt ist.

8. Verfahren nach einem der vorhergehenden Ansprüche, **dadurch gekennzeichnet**, dass die Metathesis bzw. Umsetzung mit einem Katalysator im beweglichen Bett durchgeführt wird.

9. Verfahren nach Anspruch 1, **dadurch gekennzeichnet**, dass das Isobuten und das 1-Buten in dem C₄-Schnitt wenigstens teilweise aus dem olefinischen C₅-Schnitt stammen können, der einer Behandlung unterzogen wird, die we nigstens zwei der folgenden nacheinander ablaufenden Stufen aufweist:
1) selektive Hydrierung der Diolefine und der acetylenischen Verunreinigungen mit gleichzeitiger Isomerisierung der alpha-C₅-Olefine zu 2-Pentenen und zu 2-Methyl-2-Buten durch Passieren dieses C₅-Schnittes in flüssiger Phase über einen Katalysator, der wenigstens ein aus der durch Nickel, Palladium und Platin gebildeten Gruppe gewähltes Metall umfasst, das auf einem Träger bei einer Temperatur von 20 - 200°C, einem Druck von 1 - 5 MPa, einer Raumgeschwindigkeit von 0,5 - 10 h⁻¹ abgeschieden ist, mit einem (molaren) Verhältnis H₂/Olefine von 0,5 bis 5 in einer Weise, um einen Abstrom zu erhalten, der hauptsächlich 2-Penten und 2-Methyl-2-Buten enthält und praktisch weder Diolefine noch acetylenische Verbindungen enthält,
2) Metathesis bzw. Umsetzung wenigstens eines Teils des aus der vorhergehenden Stufe stammenden Abstroms mit Ethylen in Gegenwart eines Katalysators, der wenigstens ein auf einem Träger abgeschiedenes Oxid von Rhenium umfasst bei einer Temperatur, die zwischen 0 und 100°C liegt, und einem Druck, der wenigstens gleich der Dampfspannung der Reaktionsmischung bei der Reaktionstemperatur ist, in einer Weise, um einen Abstrom zu erhalten, der grundsätzlich Propylen, 1-Buten und Isobuten enthält, wobei die Metathesis bzw. Umsetzung von einer Trennung des Propylens gefolgt ist,
3) Einführung der Mischung 1-Buten und Isobuten in eine Hydrierungsstufe des C₄-Schnittes.

10. Verfahren nach einem der vorhergehenden Ansprüche, **dadurch gekennzeichnet,** dass die C₄- und C₅-Schnitte aus einer Dampfcrackeinheit stammen.

11. Verfahren nach Anspruch 9, **dadurch gekennzeichnet**, dass die einzige Metathesis bzw. Umsetzungsstufe gleichzeitig den Hydroisomerisierungsabstrom des C₅-Schnittes und des verbleibenden C₄-Schnittes behandelt.

12. Anlage für die Umwandlung eines olefinischen C₄-Schnittes zu Polyisobuten und zu Propylen, **dadurch gekennzeichnet**, dass sie aufeinanderfolgend umfasst:
1) eine selektive Hydrierungszone 1 mit gleichzeitiger Isomerisierung des 1-Butens zu 2-Butenen, wobei diese Zone wenigstens ein Mittel 5 zur Einführung des umzuwandelnden Schnittes, wenigstens ein Mittel 11 zum Austritt des Abstroms, wenigstens ein Mittel 6 zur Einführung des Wasserstoffs in diese Zone umfasst, wobei diese Zone ebenso wenigstens ein Katalysatorbett umfasst,
2) eine Polymerisations- und Trennzone 2, die wenigstens ein Mittel zur Einführung des Isobutens des aus der Zone 1 stammenden Abstroms, wenigstens ein Mittel 8 zum Austritt des Polymers, wenigstens ein Mittel 13 zum Austritt des restlichen C₄-Schnittes ohne Polymer umfasst, wobei die Polymerisationszone wenigstens einen sauren Katalysator enthält,
3) eine Metathesis bzw. Umsetzungszone 3, die wenigstens einen Katalysator gefolgt von einer Trennzone umfasst und wenigstens ein Mittel 13 zur Einführung des restlichen C₄-Schnittes, wenigstens ein Mittel 7 zur Einführung von Ethylen und wenigstens ein Mittel 10 zum Austritt von Propylen und wenigstens ein Mittel 9 zum Austritt der Nebenprodukte umfasst.

13. Anlage nach Anspruch 12, **dadurch gekennzeichnet,** dass die Zone 2 einer Extraktionszone von Isobuten vorangestellt ist und, dass sie eine Polymerisationszone, gefolgt von wenigstens einer Trennzone umfasst.

14. Anlage nach einem der Ansprüche 12 oder 13, **dadurch gekennzeichnet**, dass die Zone 2 eine Polymerisationszone, gefolgt von wenigstens einer Trennzone umfasst.

15. Anlage nach Anspruch 11, **dadurch gekennzeichnet**, dass die Zone 3 ein bewegliches Katalysatorbett umfasst.

16. Anlage nach einem der vorhergehenden Ansprüche, **dadurch gekennzeichnet**, dass sie auch einen Abschnitt zur Behandlung eines olefinischen C₅-Schnittes umfasst, um einen Teil wenigstens des umzuwandelnden C₄-Schnittes zu erzeugen, wobei dieser C₅-Schnitt insbesondere Diolefine, n-Pentene, Methylbutene und acetylenische Verunreinigungen enthalten kann, die nacheinander wenigstens umfasst:
1) eine selektive Hydrierungszone 21 mit gleichzeitiger Isomerisierung der alpha-C₅-Olefine mit 5 Kohlenstoffatomen zu 2-Pentenen und zu 2-Methyl-2-Buten, wobei diese Zone wenigstens ein Mittel 20 zur Einführung des C₅-Schnittes, wenigstens ein Mittel 23 zum Austritt des Abstroms, wenigstens ein Mittel 22 zur Einführung des Wasserstoffs in diese Zone umfasst, wobei diese Zone ebenso wenigstens ein Katalysatorbett umfasst,
2) eine Metathesis- bzw. Umsetzungszone 24, die wenigstens ein Mittel 23 zur Einführung Abstroms aus Zone 21, wenigstens ein Mittel 25 zur Einführung von Ethylen, wenigstens ein Mittel 26 zum Austritt des Propylens, wenigstens ein Mittel 27 zum Austritt der Nebenprodukte und wenigstens ein Mittel 28 zum Austritt einer Mischung von 1-Buten und Isobuten und die in die Zone 1 geführt wird, umfasst.

17. Anlage nach einem der Ansprüche 12 bis 16, **dadurch gekennzeichnet**, dass das (die) Mittel zur Einführung des umzuwandelnden Schnittes mit einer Dampfcrackzone verbunden ist, die einen C₄- oder C₅-Schnitt erzeugt, und dass das Mittel zur Einführung von Ethylen in die Metathesis bzw. Umsetzungszone mit dieser Dampfcrackzone verbunden ist.
